# EUROPEAN PATENT SPECIFICATION

(11) **EP 0 553 126 B1**
(45) Date of publication and mention of the grant of the patent: **23.08.1995**
(21) Application number: 91917299.9
(22) Date of filing: 08.10.1991
(51) Int. Cl.: C13K 3/00

(54) **PROCESS FOR PRODUCING GLUCOSE AND FRUCTOSE FROM SUCROSE**
VERFAHREN ZUR HERSTELLUNG VON GLUKOSE UND FRUKTOSE AUS SUKROSE
PROCEDE SERVANT A PRODUIRE DU GLUCOSE ET DU FRUCTOSE A PARTIR DE SUCROSE

(30) Priority: 15.10.1990 FI 905069
(43) Date of publication of application: 04.08.1993
(73) Proprietor: XYROFIN OY, 00240 Helsinki (FI)
(72) Inventor: HEIKKILÄ, Heikki, SF-00230 Espoo (FI); HYÖKY, Göran, SF-02460 Kantvik (FI); NIITTYMÄKI, Pentti, SF-48300 Kotka (FI); VILJAVA, Tapio, SF-02460 Kantvik (FI); MYÖHÄNEN, Tuula, NL-2343 XX Oegstgeest (NL)
(74) Representative: Hansen, Bernd, Dr. Dipl.-Chem.
(86) International application number: FI9100309
(87) International publication number: WO9207097

(56) References cited:
- WO-A-91/04342
- US-A- 3 692 582
- US-A- 4 267 054
- US-A- 4 332 623
- US-A- 4 379 751
- US-A- 4 472 203
- Dialog Information Services, File 357, Biotechnology Abstract, Accession No.84-00674, HASHIMOTO K et al: "A new process combining adsorption and enzymereaction for producing higher-fructose syrup - using immobilized glucose-isomerase; mathematical model", & Chem.Pharm.Bull. (25, 10, 2371-93) 1983.
- Journal of Food Engineering, Vol. 8, 1988 K HASHIMOTO et al.: "ContinuousSeparation of alpha-Cyclodextrin and Glucose Using a Simulated Moving-BedAdsorber", see page 187 - page 200.
- Enzyme Microb. Technol., Vol. 3, October 1981 J HRADIL et al.: "Inversion ofsucrose with alpha-D-fructofuranosidase (invertase) immobillied on bead DEAHP-cellulose: batch process", see page 331.

## Description

The invention relates to an improved process for producing glucose and fructose from sucrose. The process comprises the hydrolysis of sucrose by an immobilized enzyme and the separation of glucose and fructose from the hydrolysis product chromatographically by utilizing a simulated moving bed.

Traditionally, glucose and fructose have been produced from sucrose solutions or syrups by hydrolyzing sucrose by an acid catalysis (e.g. by using a cation exchanger in acid form) or enzymatically by the invertase enzyme. The separation of glucose and fructose from the hydrolyzed solution has been carried out by chromatographic processes, which has been problematic in that the separation has taken place as a batch process, which is difficult to apply on industrial scale, and the dry matter contents of the obtained glucose and fructose solutions have been low.

U.S. Patent 3,692,582, for instance, discloses a method in which glucose and fructose are produced from sucrose as a batch process. In this patent specification, sucrose is hydrolyzed by utilizing an acid catalysis or a cation exchanger in acid form. The obtained hydrolysis product is treated by a batch process chromatographically by using a cation exchanger in calcium form, preferably a polystyrene sulphonate-based cation exchanger crosslinked by divinyl benzene, as column packing. In this patent, the highest dry matter contents of the obtained fructose fraction are 12 g/100 ml.

British Patent Specification 1 085 696 discloses a batch process comprising the hydrolysis of sucrose and the separation of glucose and fructose fractions in one and the same column packed with a cation exchanger partially in acid form and partially in calcium form (sulphonated polystyrene-based resin crosslinked by divinyl benzene). The process of this patent specification provides an effective separation of glucose and fructose, the highest dry matter contents of the obtained glucose and fructose fractions being reported to be 141 g/l and 132 g/l.

Barker, P.E. and Chuach, C.H. (*The Chemical Engineer,* August/September 1981, p. 389-393) describe a semi-continuous chromatographic process for the separation of fructose and glucose from a solution containing 50% by weight of solids (50/50% of fructose and glucose), whereby the dry matter contents of the obtained solutions were more than 10% by weight for the glucose fraction and more than 4.1% by weight for the fructose fraction.

A recent development in the separation technology is simulated moving-bed chromatography in which the object is to provide a continuous separation process with a high degree of separation and, at the same time, increased dry matter contents for the solutions in order to reduce the concentration steps.

Finnish Patent Application 882740 discloses a simulated moving-bed process for fractionating molasses into betaine, sucrose and residual molasses fractions.

Continuous chromatographic processes for separating glucose and fructose from sugar-containing solutions are described e.g. in U.S. Patents 4,157,267 (zeolite as the stationary phase); 4,267,054 (an ion exchange resin, such as Duolite C-20X4 cation exchanger in calcium form, as the stationary phase); 4,332,623 (e.g. a strongly acid cation exchanger Diaion FRK-01 in calcium form as the stationary phase); 4,379,751 (a cation exchanger in calcium form, such as Duolite C-20X4, as the stationary phase). The processes described in these patent documents are reported to provide glucose and fructose solutions having dry matter contents as high as 20% and more, up to nearly 30%.

When the separation is carried out continuously by utilizing a simulated moving bed, impurities contained in the hydrolysis solution tend to deteriorate the separation result. It is known that an acid hydrolysis of sucrose always results in an impure solution containing disaccharides of different types in addition to glucose and fructose. An enzymatic hydrolysis is more specific than an acid hydrolysis and only small quantities of undesired impurities are formed. In order that the hydrolysis could be carried out as a continuous process, it is necessary to immobilize the enzyme on a suitable carrier. Promising results have been obtained when DEAHP cellulose has been used as a carrier [Hradil, J. & Svek F., *Enzyme Microb. Technol.,* 3 (1981) 331 - 335]. The used carrier is, however, relatively soft, wherefore it is not particularly suitable for a large-scale industrial process.

U.S. Patents 4,110,164 and 4,168,250 describe ion exchange resins suitable for use as carriers in the immobilization of macromolecules, such as glucose-isomerase (an enzyme isomerizing glucose into fructose). Such ion exchange resins are prepared from an agglomerated cellulose derivative strengthened by polystyrene and derivatized utilizing e.g. carboxymethyl or diethylaminoethyl groups.

The object of the present invention is to provide a process of producing glucose and fructose from sucrose-containing solutions with high yields and with a sufficiently high degree of purity in such a way that both the hydrolysis and the separation of the fractions from each other can be carried out as a continuous process and that the concentrations of the products in the solutions obtained from the separation process are sufficiently high from the economic point of view.

This object is achieved according to the invention by combining enzymatic hydrolysis with simulated-bed chromatography, the hydrolysis step being carried out by using the invertase enzyme immobilized on a cellulose-based carrier.

The combining of a continuous enzymatic hydrolysis with continuous simulated moving-bed chromatography in accordance with the invention involves a considerable improvement over the known batch process in the production of glucose and fructose from sucrose, and it provides glucose and fructose of a very high degree of purity with high yields.

The process according to the invention comprises a hydrolysis step in which sucrose in an aqueous solution is hydrolyzed by an invertase enzyme immobilized on an agglomerated cellulose derivative to obtain a hydrolysis solution containing substantially pure fructose and glucose, and a separation step in which a glucose fraction and a fructose fraction are separated from the hydrolysis solution by utilizing simulated moving-bed chromatography.

The hydrolysis step is carried out continuously by feeding sucrose solution through a column packed with the immobilized enzyme. The flow can be effected by means of gravity or by means of a pump. The sucrose content of the solution to be fed into the column generally ranges from 30 to 60% by weight.

The carrier of the invertase enzyme is an agglomerated cellulose-based resin strengthened by polystyrene. It is mechanically strong and chemically resistant and its particle size is also suitable for use on industrial scale. One suitable carrier is diethylaminoethyl (DEAE) cellulose, prepared as described e.g. in U.S Patents 3,823,133, 4,110,164 and 4,168,250.

Temperature is preferably about 50°C at the hydrolysis step.

Sucrose is hydrolyzed at the hydrolysis step substantially completely into glucose and fructose. The glucose and fructose fractions are separated from the obtained hydrolysis solution by utilizing a simulated moving-bed chromatographic system; such a process is described e.g. in Finnish Patent Application 882740.

This separation process utilizes several chromatographic columns in a series. The series may comprise 2 to 14 columns. The columns are interconnected by means of pipelines so that recycling through all the columns can be effected with a single pump. In place of a single pump, several pumps can be used; for instance, pumps can be provided between some or all of the columns. The flow rate is 2 to 5 m³ per hour and per square metre of the cross-section of the column.

The columns are provided with feed lines and product lines for feeding eluant water and feed solution into the column and for collecting a fraction from the column, respectively. The feed and product lines are provided with valves, and so the feeding and collecting can be ended after a preselected amount of solution has been fed or collected. The pipelines interconnecting the columns are also provided with valves. The feed lines are further provided with pumps for pumping eluant water and feed solution into the preselected columns; the pipelines also comprise heat exchangers. The product lines are provided with on-line instruments for monitoring the quality of the product during operation. Such on-line instruments include a flow meter, a density meter, a refractive index detector, a conductivity meter, optical activity meter, thermometer, etc. A microprocessor or a computer is used for process control.

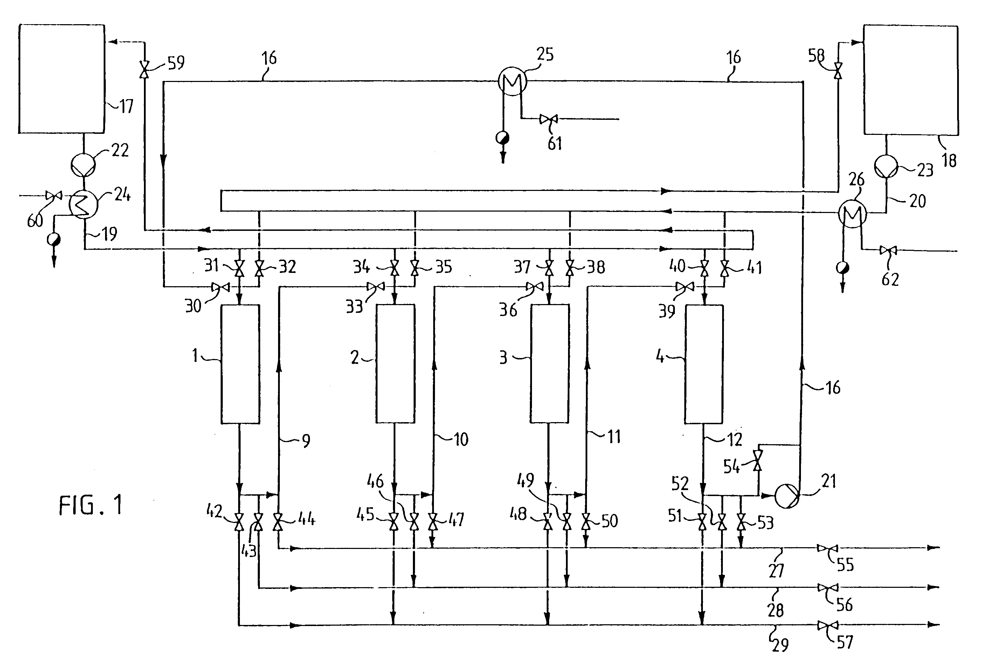

Figure 1 shows schematically a specific equipment suitable for use in the separation process. The equipment comprises columns 1 to 4 connected in a series; pipelines 9 to 16 interconnecting the columns; a container 17 for hydrolysis solution; a water tank 18; an inlet pipe 19 for the hydrolysis solution; a water inlet pipe 20; a circulation pump 21; an inlet pump 22 for the hydrolysis solution; a water inlet pump 23; heat exchangers 24 to 26; outlet pipes 27 to 29 for the product fractions; and valves 30 to 62.

Figure 2 shows schematically an equipment comprising eight columns 1 to 8.

The columns are packed with strongly acid geltype cation exchanger (such as Finex V09 C; manufacturer Cultor Ltd.), preferably in calcium form.

The feed solution is a hydrolysis solution obtained from the hydrolysis step and usually diluted to a dry matter content of 50 to 60% by weight with water or with a solution recycled from the separation step, and it contains glucose and fructose and very small amounts of oligosaccharides as solutes. The temperature of the feed solution is adjusted to 40 to 85°C before feeding into the separation system.

The temperature of the water used in elution is preferably 60 to 70°C.

During separation a batch of the feed solution is recycled by means of the pumps through the column series. A new feed solution batch is added to the column series between the oligosaccharide fraction and the fructose fraction to the top of a preselected column. While the feed solution is admitted into one column, glucose fraction is simultaneously collected from the bottom of another column. At the same time fructose is eluted from still another column while eluant water is added so that the amount of solution in the system remains constant.

The dry matter contents of the glucose and fructose fractions obtained by the process of the invention are between 25 and 33% by weight, and the glucose and fructose are in substantially pure form (>95%) in the obtained fractions. The glucose and fructose can be advantageously crystallized from the fractions with a high yield.

The process of the invention will be illustrated by means of the following examples, which are not intended to restrict the scope of the invention.

In the examples, the enzyme activities are given in Sumner units (SU). One Sumner unit is the amount of enzyme which forms 1 mg of invert sugar in 5 minutes at pH 4.5 and 20.0°C in standard conditions (5 ml of 6.5% sucrose solution + 1 ml of enzyme solution).

### Example 1

### Immobilization of invertase

Agglomerated diethylaminoethyl cellulose strengthened by polystyrene, prepared, e.g., as described in U.S. Patents 3,823,133, 4,110,164 and 4,168,250, was used as the carrier for the invertase. 200 g of this DEAE cellulose carrier was submerged in water, heated to 80°C and allowed to stand overnight at room temperature. The carrier was filtered from the water by suction using a fritted glass funnel.

The used enzyme was a commercial product of Honeywill and Stein Ltd., the enzyme activity of which is stated to be 6,260 Sumner units/ml. 250 ml of invertase concentrate (enzyme activity 1,565,000 SU) was diluted with water at a ratio of 1 : 1, mixed with the above-mentioned 200 g of carrier, and allowed to stand at 45°C for 4 hours under mixing.

The pH of the resulting solution was 7.0 and conductivity 1,000 »S/cm.

The enzyme activity bound to the carrier was 1,354,000 SU. Thus the activity of the enzyme immobilized in the system was 6,770 SU per one gram of the carrier material. The immobilized enzyme was stored in 55% glycerol at pH 5.8.

### Hydrolysis (inversion)

20 ml of the immobilized enzyme prepared as described above was packed into a column (1.6 x 10 cm) and sucrose solution containing sucrose 620 g/l was pumped through the column at pH 5.0 and 50°C. The flow rate was 40 ml per hour.

It was observed that the degree of inversion was more than 99%. The composition of the solution leaving the column was as follows

| Concentration, % on dry matter | | | |
|---|---|---|---|
| Glucose | Fructose | Sucrose | Other disaccharides |
| 50.0 | 49.3 | 0.7 | - |

### Example 2

The same carrier as in Example 1 was used in the immobilization of the enzyme. 400 kg (1,000 l) of the carrier was slurried in water in a 2,000 litre tank, in which it was allowed to absorb water overnight. The carrier was decanted five times to remove fine solids by using one carrier volume of water each time.

500 kg of an invertase enzyme of the same type as in Example 1 and having an activity of 5,498 SU/ml, specific weight 1.14 kg/l, pH 4.8 and conductivity 1,050 »S/cm was poured into the tank, which contained the carrier. The mixture was mixed for one hour. The pH of the resulting suspension was 7.2 and conductivity 800 »S/cm. The tank was filled with hot water in such a way that the temperature of the tank was 45°C (altogether about 1,000 l) and the mixing was continued for 4 hours.

The activity of the carrier loaded with the enzyme was 6,030 SU/g of the carrier.

Water was displaced from the tank by pumping sucrose solution containing 620 g/l sucrose into the tank through a bottom valve. The carrier/ sucrose slurry was pumped into a column having a volume of 2 m³ and a diameter of 1 m.

Inversion was carried out by pumping sucrose solution through the column at 50°C and pH 5.0. The solution leaving the column contained 49.2% of fructose, 50.5% of glucose and 0.3% of sucrose on dry matter basis.

### Chromatography

The fructose-glucose solution obtained above was separated into fructose and glucose fractions by a continuous process by utilizing a simulated moving-bed system.

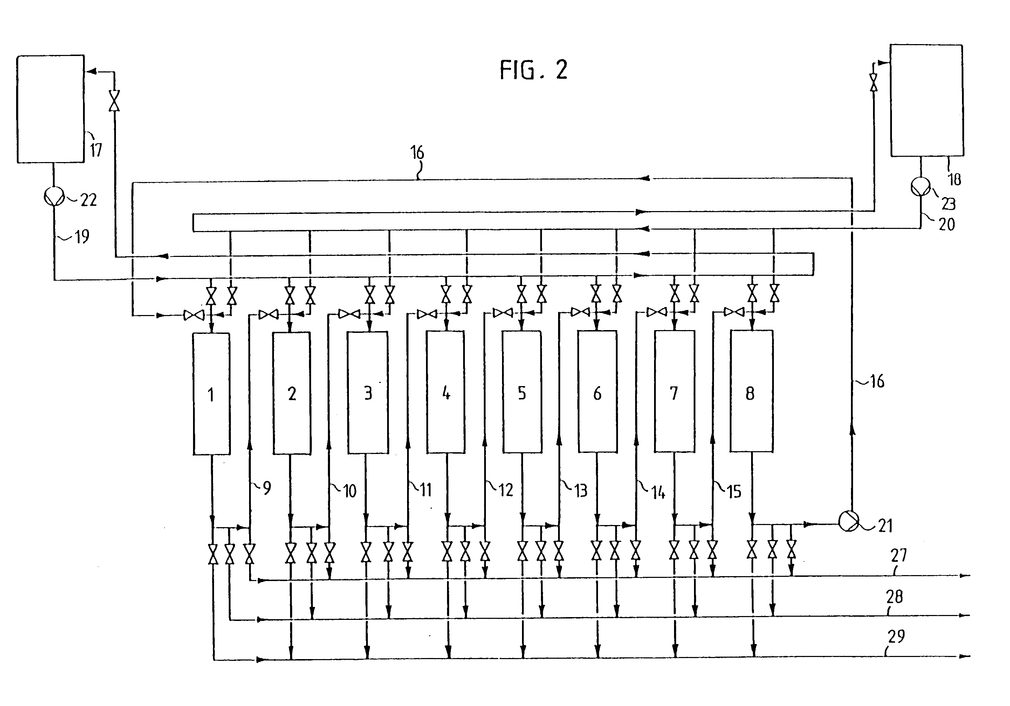

A pilot plant scale test equipment was used. It comprised 8 columns, an inlet pump, a circulation pump, an eluant water pump, flow and pressure regulators, and inlet and outlet valves for different process streams. The flow sheet is shown in Figure 2.

The diameter of each column was 0.2 m and the height of the packing material in each column was 1.25 m. The packing material was a strongly acid cation exchanger Finex V09 C (manufacturer Cultor Ltd.); sulphonated polystyrene; crosslinked by divinyl benzene, 5.5%; a mean particle size was 0.36 mm. The packing material had been regenerated into calcium form.

The flow rate was 120 l/h and temperature 65°C.

The separation of fructose and glucose was carried out by a 5-step sequence. The columns were connected in a series and the direction of flow was always from the column 1 to the column 2, and so on, and from the column 8 back to the column 1, as follows:
- Step 1:: Hydrolysis solution was fed into the column 1 and glucose fraction was collected from the column 2. Simultaneously water was fed into the column 5 and fructose fraction was collected from the column 6.
- Step 2:: Glucose fraction was still collected from the column 2, simultaneously feeding water into the column 5.
- Step 3:: Recycling of the solutions in all the columns by the circulation pump.
- Step 4:: Water was fed into the column 3 and oligosaccharide fraction was collected from the column 2.
- Step 5:: Recycling of the solutions in all the columns by the circulation pump.

After the sequence had been completed, the process control program continued by returning to step 1. By repeating this sequence 7 or 8 times, the system was equilibrated so that the compositions of the eluted product fractions were constant.

The separation was controlled by a microprocessor, which defined the accurately predetermined volumes of feed, recycling and product fractions. The volumes are shown in the following Table 1:

**Table 1**

| Volume (l) | | | | | |
|---|---|---|---|---|---|
| Step No. | 1 | 2 | 3 | 4 | 5 |
| Feed | 30 | - | - | - | - |
| Fructose fraction | 30 | - | - | - | - |
| Glucose fraction | 30 | 3 | - | - | - |
| Oligosaccharide fraction | - | - | - | 15 | - |
| Recycling | - | - | 147 | - | 15 |

The purity of the obtained fructose fraction was 96.9% and its concentration was 30.0% by weight. The yield was 95% from the feed. Sucrose residues were separated completely from the fructose. The fructose fraction contained 2.6% of glucose and 0.7% of oligosaccharides.

### Example 3

A saccharide solution was hydrolyzed by an immobilized enzyme substantially as described in Examples 1 and 2. In this way 2.6 m³ of hydrolysis solution was prepared the dry matter content of which was 650 g/l, of which 47% was fructose, 52% glucose and 1% oligosaccharides.

The separation was carried out by simulated moving-bed chromatography substantially similarly as in Example 2 by using four interconnected columns, each with a volume of 22 m³.

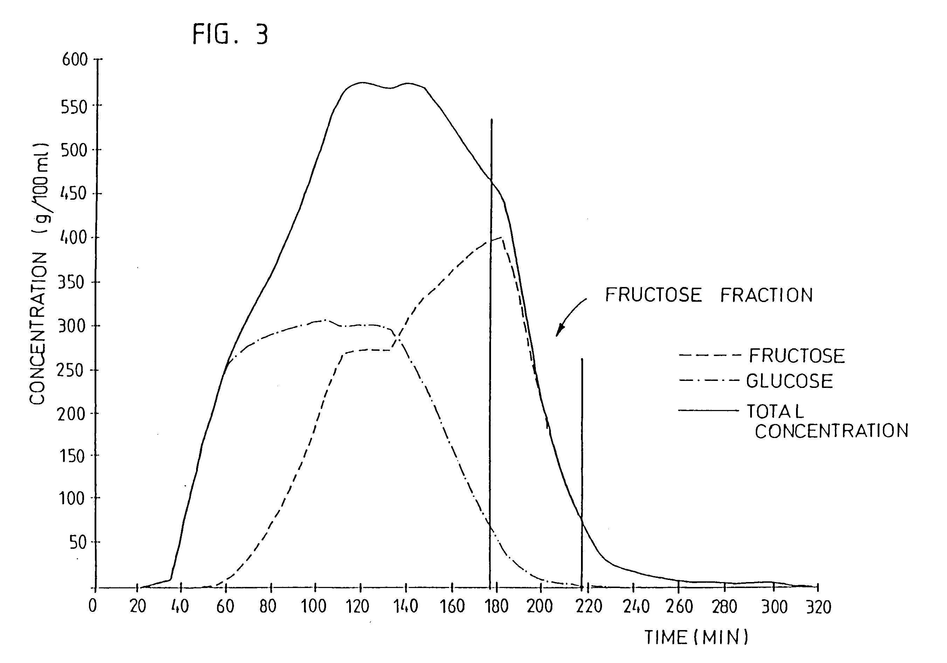

The obtained fructose fraction contained 96% of fructose and 4% of glucose on dry matter basis, and no sucrose. The elution of glucose and fructose appears from Figure 3, in which the points between which the fructose fraction was recovered are indicated by division lines.

### Example 4

From the glucose and fructose fractions obtained in Example 2, fructose can be crystallized e.g. in a manner described in U.S. Patent 3,883,365 and glucose can be crystallized as described in Dean, G.R. and Gottfried, J.B., Adv. Carboh. Res. 5 (1950) p. 127-142; and Kirk-Othmer, *Encyclopedia of Chem. Tech.,* vol. 6, p. 924.

### A. Fructose

Fructose was crystallized by a two-step cooling crystallization similarly as described in Example IV of U.S. Patent 3,883,365. The solution was adjusted to pH 5.5 and evaporated to a dry matter content of 92.4% by weight. The crystallization time was 60 hours + 70 hours and the yield of crystalline fructose was 43% from the dry matter.

### B. Glucose

The glucose solution was evaporated to a dry matter content of 70 to 80% by weight, cooled and fed into crystallizers. The crystallizer is usually a horizontal cylindrical tank provided with a slow mixer, a cooling-water jacket and cooling spirals. Seed crystals were introduced into the crystallizer (20 to 25% of the preceding crystallization was left in the crystallizer) and glucose solution at about 46°C was mixed with the seeds, so that the initial temperature was about 43°C. The mass was cooled slowly to 20 to 30°C during 3 to 5 days; about 60% of the glucose was thereby crystallized into monohydrate crystals, which were separated. Thereafter the wet glucose was dried.

## Claims

1. A process for producing glucose and fructose from sucrose by an enzymatic hydrolysis, **characterized** in that sucrose solution is hydrolyzed as a continuous process by an invertase enzyme immobilized on a solid carrier, and a glucose fraction and fructose fraction are separated from the solution obtained as a result of the hydrolysis as a continuous process by a chromatographic simulated moving-bed process.

2. A process according to claim 1, **characterized** in that the carrier of the invertase enzyme is an agglomerated cellulose resin strengthened by polystyrene and derivatized by diethylaminoethyl groups.

3. A process according to claim 1 or 2, **characterized** in that the chromatographic simulated moving-bed system comprises at least 2 chromatographic columns in a series and the flow of liquid takes place in one direction in the columns, and that glucose and fructose are separated during the same sequence, said sequence comprising the steps of
a) feeding hydrolysis solution, whereby a glucose- and fructose-containing solution obtained from the hydrolysis of the sucrose solution is fed into one of said columns while substantially simultaneously eluting a glucose fraction from a bottom of a column, said bottom being positioned in the column system downstream of the top of the column to which said feed solution is fed, and while simultaneously feeding water into a column positioned in the column system downstream of the column from which glucose is eluted, and fructose is eluted from a bottom of a column, said bottom being positioned downstream of the top of the column to which water is fed,
b) further eluting the glucose fraction, whereby the elution of the glucose fraction from the bottom of the same column as above and the feeding of eluant water into the same column as above are continued,
c) recycling the solutions contained in the system through all said columns,
d) eluting an oligosaccharide fraction from one column while feeding eluant water into a column downstream of said column, and
e) further recycling the solutions contained in the system through said columns,
and that the sequence comprising the steps a) to e) is repeated until equilibrium is obtained.

4. A process according to claim 1 or 2, **characterized** in that the chromatographic simulated moving-bed system comprises at least 2 chromatographic columns in a series and the flow of liquid takes place in one direction in the columns and that glucose and fructose are separated during the same sequence comprising the steps of
a) feeding hydrolysis solution, whereby a glucose- and fructose-containing solution obtained from the hydrolysis of the sucrose solution is fed into one of said columns while substantially simultaneously eluting a glucose fraction from a bottom of a column, said bottom being positioned in the column system downstream of the top of the column to which said feed solution is fed, and while simultaneously feeding water into a column positioned in the column system downstream of the column from which glucose is eluted, and fructose is eluted from a bottom of a column, said bottom being positioned downstream of the top of the column to which water is fed,
b) further eluting the glucose fraction, whereby the elution of the glucose fraction from the bottom of the same column as above and the feeding of eluant water into the same column as above are continued,
c) recycling the solutions contained in the system through all said columns,
and that the sequence comprising the steps a) to c) is repeated until equilibrium is obtained.

5. A process according to claim 3 or 4, **characterized** in that the number of chromatographic columns in the series is from 2 to about 14, preferably from 4 to 8.

6. A process according to any one of claims 3 to 5, **characterized** in that the stationary phase of the chromatographic columns is a strongly acid cation exchange resin.

7. A process according to claim 6, **characterized** in that the strongly acid cation exchange resin is in calcium form.

8. A process according to any one of claims 3 to 7, **characterized** in that the linear flow rate of liquid in the columns is between 2 and 5 m/h.

9. A process according to any one of claims 3 to 8, **characterized** in that the temperature of the feed solution and eluant water to be recycled is between about 40°C and about 85°C.

## Patentansprüche

1. Verfahren zur Herstellung von Glukose und Fruktose aus Saccharose durch enzymatische Hydrolyse, dadurch **gekennzeichnet,** daß die Saccharoselösung in einem kontinuierlichen Verfahren durch ein Invertase-Enzym, das auf einem festen Träger immobilisiert ist, hydrolysiert wird, und eine Glukosefraktion und eine Fruktosefraktion von der als Ergebnis der Hydrolyse erhaltenen Lösung in einem kontinuierlichen Verfahren durch ein chromatographisches simuliertes Fließbettverfahren abgetrennt werden.

2. Verfahren nach Anspruch 1, dadurch **gekennzeichnet,** daß der Träger des Invertase-Enzyms ein durch Polystyrol verfestigtes und durch Diethylaminoethylgruppen derivatisiertes agglomeriertes Zelluloseharz ist.

3. Verfahren nach Anspruch 1 oder 2, dadurch **gekennzeichnet,** daß das chromatographische simulierte Fließbettsystem mindestens 2 chromatographische Säulen in Serie umfaßt, und der Fluß der Flüssigkeit in einer Richtung in den Säulen stattfindet, und daß die Glukose und Fruktose während derselben Sequenz getrennt werden, wobei die Sequenz die Schritt umfaßt:
a) Zuführen von Hydrolyselösung, wodurch eine Glukose- und Fruktose-haltige Lösung, erhalten aus der Hydrolyse der Saccharoselösung, einer der Säulen zugeführt wird, während im wesentlichen gleichzeitig eine Glukosefraktion vom Boden einer Säule eluiert wird, wobei der Boden in der Säule im Säulensystem stromabwärts von der Spitze der Säule, in welche die Zuführlösung eingeleitet wird, positioniert ist, und während gleichzeitig Wasser in eine Säule, die im Säulensystem stromabwärts der Säule, von der Glukose eluiert wird, angeordnet ist, eingeleitet wird, und Fruktose vom Boden einer Säule eluiert wird, wobei die Säule stromabwärts der Spitze der Säule, der das Wasser zugeführt wird, positioniert ist,
b) weitere Elution der Glukosefraktion, wodurch die Elution der Glukosefraktion vom Boden derselben Säule wie oben und Zuführen von Eluatwasser in dieselbe Säule wie oben fortgesetzt wird,
c) Zurückführen der in dem System enthaltenen Lösungen durch alle Säulen,
d) Eluieren der Oligosaccharidfraktion von einer Säule, während Eluatwasser in eine Säule stromabwärts der Säule zugeführt wird, und ferner
e) weitere Rückführung der in dem System enthaltenen Lösungen durch die Säulen,
und daß die Sequenz die Schritte a) bis e) wiederholt wird, bis ein Gleichgewicht erreicht ist.

4. Verfahren nach Anspruch 1 oder 2, dadurch **gekennzeichnet,** daß das chromatographische simulierte Fließbettsystem mindestens 2 chromatographische Säulen in Serie umfaßt, und daß der Flüssigkeitsfluß in einer Richtung in den Säulen stattfindet, und daß die Glukose und Fruktose während der gleichen Sequenz umfassend die Schritte aufgetrennt werden:
a) Zuführen von Hydrolyselösung, wodurch eine Glukose- und Fruktose-haltige Lösung, erhalten aus der Hydrolyse der Saccharoselösung, einer der Säulen zugeführt wird, während im wesentlichen gleichzeitig Glukosefraktion vom Boden einer Säule eluiert wird, wobei der Boden im Säulensystem stromabwärts von der Spitze der Säule, der die Zuführlösung zugeführt wird, angeordnet ist, und während gleichzeitig Wasser in eine Säule zugeführt wird, die im Säulensystem stromabwärts von der Säule, von der Glukose eluiert wird, positioniert ist, und Fruktose vom Boden einer Säule eluiert wird, wobei der Boden stromabwärts der Spitze der Säule, der das Wasser zugeführt wird, angeordnet ist,
b) weiteres Eluieren der Glukosefraktion, wodurch die Elution der Glukosefraktion vom Boden derselben Säule wie oben und Zuführen von Eluatwasser in die gleiche Säule wie oben fortgesetzt wird,
c) Zurückführen der in dem System enthaltenen Lösungen durch alle Säulen,
und daß die Sequenz, umfassend die Schritte a) bis c), bis zur Einstellung eines Gleichgewichts wiederholt wird.

5. Verfahren nach Anspruch 3 oder 4, dadurch **gekennzeichnet,** daß die Anzahl der chromatographischen Säulen in der Serie von 2 bis ca. 6 ist, vorzugsweise von 4 bis 8.

6. Verfahren nach einem der Ansprüche 3 bis 5, dadurch **gekennzeichnet,** daß die stationäre Phase der Chromatographiesäulen ein stark saures Kationenaustauscherharz ist.

7. Verfahren nach Anspruch 6, dadurch **gekennzeichnet,** daß das stark saure Kationenaustauscherharz in einer Kalziumform vorliegt.

8. Verfahren nach einem der Ansprüche 3 bis 7, dadurch **gekennzeichnet,** daß die lineare Flußrate der Flüssigkeit in den Säulen zwischen 2 und 5 m/h ist.

9. Verfahren nach einem der Ansprüche 3 bis 8, dadurch **gekennzeichnet,** daß die Temperatur der Zuführlösung und des Eluatwassers, das wieder zugeführt wird, ca. zwischen 40°C und ca. 85°C ist.

## Revendications

1. Procédé de préparation de glucose et de fructose à partir de saccharose au moyen d'une hydrolyse enzymatique, caractérisé en ce que la solution de saccharose est hydrolysée sous forme de procédé continu au moyen d'une enzyme invertase immobilisée sur un substrat solide, et une fraction glucose et une fraction fructose sont séparées de la solution résultant de l'hydrolyse, sous forme de procédé continu, au moyen d'un procédé chromatographique à lit mobile simulé.

2. Procédé selon la revendication 1, caractérisé en ce que le substrat de l'enzyme invertase est une résine de cellulose agglomérée, renforcée par du polystyrène et dérivatisée au moyen de groupes éthylaminoéthyle.

3. Procédé selon la revendication 1 ou 2, caractérisé en ce que le système chromatographique à lit mobile simulé comporte au moins 2 colonnes chromatographiques montées en série, et l'écoulement du liquide dans les colonnes s'effectue dans une seule direction, et en ce que le glucose et le fructose sont séparés pendant la même séquence, ladite séquence comprenant les étapes consistant à:
a) amener une solution d'hydrolyse, étape au cours de laquelle une solution contenant du glucose et du fructose, obtenue par l'hydrolyse de la solution de saccharose, est amenée dans une desdites colonnes, tout en éluant pratiquement simultanément une fraction glucose par le bas d'une colonne, ledit bas étant placé dans le système de colonnes en aval du sommet de la colonne dans laquelle ladite solution d'alimentation est amenée, et tout en amenant simultanément de l'eau dans une colonne placée dans le système de colonnes en aval de la colonne dans laquelle le glucose est élué, et le fructose est élué par le bas d'une colonne, ledit bas étant situé en aval du sommet de la colonne dans laquelle l'eau est introduite,
b) éluer ensuite la fraction glucose, tout en continuant l'élution de la fraction glucose par le bas de la même colonne que ci-dessus et d'introduire de l'eau d'élution dans la même colonne que ci-dessus,
c) recycler à travers toutes lesdites colonnes les solutions contenues dans le système,
d) éluer une fraction oligosaccharides dans une colonne, tout en introduisant de l'eau d'élution dans une colonne située en aval de ladite colonne, et
e) continuer de recycler à travers lesdites colonnes les solutions contenues dans le système,
et en ce qu'on répète la séquence comportant les étapes a) à e) jusqu'à l'obtention de l'équilibre.

4. Procédé selon la revendication 1 ou 2, caractérisé en ce que le système chromatographique à lit mobile simulé comporte au moins 2 colonnes chromatograqhiques montées en série, et l'écoulement du liquide dans les colonnes s'effectue dans une seule direction, et en ce que le glucose et le fructose sont séparés pendant la même séqence, ladite séquence comprenant les étapes consistant à:
a) amener une solution d'hydrolyse, étape au cours de laquelle une solution contenant du glucose et du fructose, obtenue par l'hydrolyse de la solution de saccharose, est amenée dans une desdites colonnes, tout en éluant pratiquement simultanément une fraction glucose par le bas d'une colonne, ledit bas étant placé dans le système de colonnes en aval du sommet de la colonne dans laquelle ladite solution d'alimentation est amenée, et tout en amenant simultanément de l'eau dans une colonne placée dans le système de colonnes en aval de la colonne dans laquelle le glucose est élué, et le fructose est élué par le bas d'une colonne, ledit bas étant situé en aval du sommet de la colonne dans laquelle l'eau est introduite,
b) éluer ensuite la fraction glucose, tout en continuant l'élution de la fraction glucose par le bas de la même colonne que ci-dessus et d'introduire de l'eau d'élution dans la même colonne que ci-dessus,
c) recycler à travers toutes lesdites colonnes les solutions contenues dans le système,
et en ce qu'on répète la séquence comportant les phases a) à c) jusqu'à l'obtention de l'équilibre.

5. Procédé selon la revendication 3 ou 4, caractérisé en ce que le nombre de colonnes chromatographiques de la série est de 2 à environ 14, de préférence de 4 à 8.

6. Procédé selon l'une quelconque des revendications 3 à 5, caractérisé en ce que la phase stationnaire des colonnes chromatographiques est une résine échangeuse de cations fortement acide.

7. Procédé selon la revendication 6, caractérisé en ce que la résine échangeuse de cations fortement acide se présente sous la forme calcium.

8. Procédé selon l'une quelconque des revendications 3 à 7, caractérisé en ce que la vitesse d'écoulement linéaire du liquide dans les colonnes est comprise entre 2 et 5 m/h.

9. Procédé selon l'une quelconque des revendications 3 à 8, caractérisé en ce que la température de la solution d'alimentation et de l'eau d'élution à recycler est comprise entre environ 40°C et environ 85°C.
